# EUROPÄISCHE PATENTSCHRIFT

(11) **EP 1 042 260 B1**
(45) Veröffentlichungstag und Bekanntmachung des Hinweises auf die Patenterteilung: **02.04.2003**
(21) Anmeldenummer: 98964487.7
(22) Anmeldetag: 04.12.1998
(51) Int. Cl.: C07C 29/149, C07C 29/17, C07C 31/20, C07C 31/27

(54) **VERFAHREN ZUR HYDRIERUNG VON CARBONSÄUREN ODER DEREN ANHYDRIDEN ODER ESTERN ZU ALKOHOLEN**
METHOD FOR HYDROGENATING CARBOXYLIC ACIDS OR THE ANHYDRIDES OR ESTERS THEREOF INTO ALCOHOL
PROCEDE D'HYDROGENATION D'ACIDES CARBOXYLIQUES OU DE LEURS ANHYDRIDES OU ESTERS EN ALCOOLS

(30) Priorität: 17.12.1997 DE 19756171
(43) Veröffentlichungstag der Anmeldung: 11.10.2000
(73) Patentinhaber: BASF AKTIENGESELLSCHAFT, 67056 Ludwigshafen (DE)
(72) Erfinder: DOSTALEK, Roman, D-67271 Neuleiningen (DE); FISCHER, Rolf, Hartmuth, D-69121 Heidelberg (DE); KRUG, Thomas, D-67549 Worms (DE); PAUL, Axel, D-68623 Lampertheim (DE); PINKOS, Rolf, D-67098 Bad Dürkheim (DE); STEIN, Frank, D-67098 Bad Dürkheim (DE)
(86) Internationale Anmeldenummer: EP9807900
(87) Internationale Veröffentlichungsnummer: WO99031035

(56) Entgegenhaltungen:
- EP-A- 0 528 305
- DE-A- 3 703 585
- GB-A- 2 215 330

## Beschreibung

Die Erfindung betrifft ein Verfahren zur Erhöhung der Katalysatoraktivität und die Vermeidung von Nebenreaktionen bei der Hydrierung von Carbonsäuren oder deren Derivaten durch Zudosierung von bestimmten basisch wirkenden Alkali oder Erdalkaliverbindungen zum Hydrierfeed.

Es ist bekannt, bei der Herstellung von Hydrierkatalysatoren basisch wirkende Komponenten mitzuverwenden. Diese sind dann z.T. noch auf dem fertigen Katalysator vorhanden. So wird z.B. in EP-A 528 305 die Herstellung eines Cu/ZnO/Al₂O₃ - Katalysators beschrieben, bei dem basische Verbindungen wie Alkalicarbonate, Alkalihydroxide oder Alkalihydrogencarbonate bei der Fällung der Katalysatorbestandteile verwendet werden. Ein kleiner Anteil des Alkalizusatzes verbleibt dann im Katalysator. Ein weiteres Beispiel zur Herstellung alkalihaltiger Katalysatoren ist in DE-A 2 321 101 beschrieben, wobei Co-haltige Katalysatoren hergestellt werden. In EP-A 552 463 ist die Herstellung von Cu/Mn/Al-Katalysatoren beschrieben, die aufgrund ihrer Herstellung ebenfalls geringe Mengen Alkali enthalten können.

GB-A-2215330 offenbart ein Verfahren zur katalytischen Hydrierung von Mono-u. Dicarbonsäureesten.

Die so hergestellten Hydrierkatalysatoren enthalten das Alkali gleichmäßig in der gesamten Katalysatormasse verteilt. Da aber immer nur die äußere bzw. zugängliche Oberfläche des Katalysators katalytisch aktiv ist, ist die Menge an Alkali an diesen Stellen sehr klein und wird durch den zu hydrierenden Strom im allgemeinen schnell ausgewaschen, da das Alkali nicht kovalent, sondern nur locker gebunden vorliegt. Ferner ist durch die Zufuhr von Verbindungen, die den Katalysator acidifizieren können, die Kapazität der durch das Alkali bedingten basischen Zentren schnell erschöpft. Damit können Reaktionen, die durch saure Katalyse gefördert werden, wie Veretherungen oder Dehydratisierungen, die Selektivität der Hydrierung zu Alkoholen mindern. Im allgemeinen wird durch die Zufuhr von Verbindungen, die den Katalysator acidifizieren, dessen Lebensdauer z.T. stark verkürzt, da sich die Struktur der Katalysatoroberfläche, z.B. durch Auswaschung von Katalysatorbestandteilen, oder deren Rekristallisation der Aktivmetalle ändert.

Die Hydrierkatalysatoren sind demgemäß z.T. empfindlich gegen den Hydrierfeed selbst bzw. gegen Verunreinigungen, die im verwendeten Wasserstoff oder im Hydrierfeed vorhanden sind. So werden beispielsweise viele Katalysatoren bei der Hydrierung von Carbonsäuren nicht nur acidifiziert, sondern auch chemisch stark geschädigt, indem Katalysatorkomponenten ausgewaschen werden. Selbst wenn keine Carbonsäuren als Einsatzmaterial hydriert werden, sondern z.B. Ester, werden üblicherweise durch Wasserspuren die Carbonsäuren durch Hydrolyse des Esters freigesetzt. Ein weiteres Problem sind Verunreinigungen, z.B. organische Halogenverbindungen, die in die Hydrierung, z.B. mit dem Wasserstoff, eingeschleppt werden können und nachteilige Folgen haben, selbst wenn diese Gehalte unter 1 ppm liegen. So ist es bekannt, daß man z.B. Cu-Katalysatoren dazu benutzen kann, Halogenspuren aus Stoffströmen durch Chemisorption zu entfernen (US 5 614 644). Ohne solch eine Vorabsorption wird das Halogen auf dem Hydrierkatalysator absorbiert und führt dort einerseits zur Acidifizierung, andererseits zu strukturellen Veränderungen des Katalysators.
Demgemäß bestand typischerweise die technische Lösung des geschilderten Problems in der Vorschaltung eines "guard-beds", in dem unerwünschte Verunreinigungen absorbiert wurden. Eine Ausschaltung der reaktionseigenen Säuren, wie bei den sich bildenden oder als Hydrierfeed eingesetzten Carbonsäuren, ist damit natürlich nicht möglich.

Es bestand daher die Aufgabe, ein Verfahren vorzuschlagen, mit dem die den Katalysator beeinträchtigende Wirkung sowohl der reaktionseigenen Säuren als auch der eingeschleppten Verunreinigungen vermindert oder ausgeschaltet werden kann.

Diese Aufgabe wurde erfindungsgemäß gelöst mit einem Verfahren zur katalytischen Hydrierung von Carbonsäuren oder deren Anhydriden oder Estern zu Alkoholen mit Wasserstoff an heterogenen Katalysatoren, die hydrierend wirkende Elemente der Gruppen 6, 7, 8, 9, 10 und 11 sowie gegebenenfalls 2, 14 und 15 des Periodensystems der Elemente enthalten, oder aus diesen bestehen, in flüssiger Phase bei Temperaturen von 100 bis 300°C und Drücken von 1-30 kPa (10 bis 300 bar), wobei der Hydrierwasserstoff und/oder der Hydrierfeed Halogenverbindungen enthalten und man in einem geeigneten Lösungsmittel als separaten Strom in die Hydrierung selbst oder in den Hydrierfeed, bezogen auf den flüssigen Hydrierfeed, 1 bis 3000 ppm, insbesondere 3 bis 1000 und bevorzugt 5 bis 600 ppm, einer basischen Alkali- oder Erdalkaliverbindung ausgewählt aus der Gruppe bestehend aus den Hydroxiden, Carbonaten, Carboxylaten und Alkoholaten zusetzt.

Aus DE 1 235 879, Beispiel 16, ist zwar schon der Zusatz von Trinatriumphosphat zum Hydrierfeed bekannt.

In diesem Beispiel ist die Hydrierung eines Carbonsäuregemisches beschrieben, das 0,1 Gew.-% Na₃PO₄, u.a. Adipinsäure, Glutarsäure, Bernsteinsäure und 6-Hydroxycapronsäure, bereits hydriertes Carbonsäuregemisch, sowie eine nicht näher definierte Menge an "rohen Monoalkoholen", deren Wassergehalt auf 7 % eingestellt ist, enthält. Der alleinige Zusatz von Na₃PO₄ zum Hydrierfeed stellt jedoch keine geeignete Maßnahme dar, um Aktivität und Standzeit des Katalysators zu erhöhen. Es tritt nach kurzer Zeit in Gegenwart acidifizierender Bestandteile des Reaktionsgemisches eine drastische Aktivitätseinbuße ein.

Carbonsäuren und Derivate können Carbonsäuren selbst und Ester, auch deren innere Ester, d.h. Lactone, und Anhydride sein. Beispiele hierzu sind Essigsäureester, Propionsäureester, Hexansäureester, Dodecansäureester, Pentadecansäureester, Hexadecansäureester, 2-Cyclododecylpropionsäureester, Ester aus Glycerin mit Fettsäuren, Maleinsäurediester, Bernsteinsäurediester, Fumarsäurediester, Glutarsäurediester, Adipinsäuredimethylester, 6-Hydroxycapronsäureester, Cyclohexandicarbonsäurediester, Benzoesäureester, Butyrolacton, Caprolacton, Maleinsäure, Bernsteinsäure, Itaconsäure, Adipinsäure, 6-Hydroxycapronsäure, Cyclohexandicarbonsäure, Benzoesäure, Maleinsäureanhydrid und Bernsteinsäureanhydrid. Bevorzugte Ausgangsmaterialien sind Diester, insbesondere Diester von niedermolekularen Alkoholen mit Dicarbonsäuren mit 4 bis 6 C-Atomen.

Als basische Verbindungen kommen vor allem Alkali- und Erdalkalimetallhydroxide, wie LiOH, NaOH, KOH, RbOH, CsOH, Mg(OH)₂, Sr(OH)₂ oder Ba(OH)₂ in Betracht. Die Alkali- oder Erdalkalimetalle können auch in Form von Carboxylaten, z.B. als Formiat, Acetat, Propionat, Maleat oder Glutarat, oder als Alkoholate wie Methanolat, Ethanolat oder Propionat vorliegen. Geeignet sind auch Carbonate. Die basische Verbindung wird in einem geeigneten Lösungsmittel als separater Strom in die Hydrierung selbst oder in den Hydrierfeed eingeschleust. Die Mengen an Alkali- bzw. Erdalkalimetall, bezogen auf den Hydrierstrom, sind sehr niedrig. Sie betragen zwischen 1 und 3000 ppm, bevorzugt zwischen 3 und 1000 ppm, besonders bevorzugt zwischen 5 und 600 ppm. Die Zufuhr der basischen Komponente erfolgt bevorzugt kontinuierlich. Es ist aber auch eine absatzweise Dosierung möglich. Dabei können innerhalb kurzer Zeit auch größere Mengen basischer Komponenten, bezogen auf den Hydrierfeed, als 3000 ppm in die Hydrierung gelangen. Im Mittel liegen die Mengen aber unter 3000 ppm.
Es ist überraschend, daß auch nach längerer Dosierung der basischen Komponenten keine Ablagerungen auf dem Katalysator auftreten.

Die Hydrierung erfolgt in der Flüssigphase. Es ist dabei nicht entscheidend, ob dabei bei fest angeordneten Katalysatoren eine Aufwärts- oder Abwärtsfahrweise gewählt wird. Ebenso möglich ist eine Hydrierung mit suspendierten Katalysatoren.
Als Hydrierkatalysatoren können im erfindungsgemäßen Verfahren im allgemeinen heterogene zur Hydrierung von Carbonylgruppen geeignete Katalysatoren Verwendung finden. Beispiele hierfür sind z.B. in Houben-Weyl, Methoden der Organischen Chemie, Band IV/1c, S. 16 bis 26, beschrieben.
Von diesen Hydrierkatalysatoren sind solche bevorzugt, die ein oder mehrere Elemente der Gruppen 6, 7, 8, 9, 10 und 11 sowie gegebenenfalls der Gruppen 2, 14 und 15 des Periodensystems der Elemente, insbesondere Kupfer, Chrom, Rhenium, Kobalt, Rhodium, Nickel, Palladium, Eisen, Platin, Indium, Zinn und Antimon, enthalten. Besonders bevorzugt sind Katalysatoren, die Kupfer, Kobalt, Palladium, Platin oder Rhenium enthalten.
Als die zu verwendenden Katalysatoren kommen vor allem sogenannte Vollkatalysatoren in Betracht. Dabei liegen die katalytisch wirkenden Metalle ganz überwiegend ohne Trägermaterialien vor. Beispiele hierzu sind Raney-Katalysatoren, z.B. auf Basis Ni, Cu oder Kobalt. Andere Beispiele sind Pd-schwarz, Pt-schwarz, Cu-Schwamm, Legierungen oder Mischungen aus z.B. Pd/Re, Pt/Re, Pd/Ni, Pd/Co oder Pd/Re/Ag.
Die im erfindungsgemäßen Verfahren eingesetzten Katalysatoren können auch z.B. sogenannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, z.B. als schwerlösliche Hydroxyde, Oxydhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C, in die entsprechenden Oxide, Mischoxide und/oder gemischtvalentigen Oxide umwandelt, die durch eine Behandlung mit Wasserstoff oder mit Wasserstoff enthaltenden Gasen bei in der Regel 50 bis 700°C, insbesondere 100 bis 400°C, zu den betreffenden Metallen und/oder oxidischen Verbindungen niederer Oxidationsstufe reduziert und in die eigentliche, katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden. Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den oben genannten Fällungskatalysatoren, welche außer den katalytisch aktiven Komponenten noch zusätzlich ein Trägermaterial enthalten, eignen sich für das erfindungsgemäße Verfahren im allgemeinen solche Trägermaterialien, bei denen die katalytisch-hydrierend wirkende Komponenten, z.B. durch Imprägnierung, auf ein Trägermaterial aufgebracht worden sind.
Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Trägermaterialien z.B. durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten, oder thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Auf dampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Jedoch führen höhere Gehalte an katalytisch aktiven Metallen in der Regel zu höheren Raum-Zeit-Umsätzen als niedrigere Gehalte. Im allgemeinen werden Trägerkatalysatoren verwendet, deren Gehalt an katalytisch aktiven Metallen 0,1 bis 90 Gew.-%, vorzugsweise 0,5 bis 40 Gew.-%, bezogen auf den gesamten Katalysator, beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindungsgemäßen Verfahrens auswirkt. Selbstverständlich können auch mehrere der katalytisch aktiven Metalle aus dem jeweiligen Trägermaterial aufgebracht sein. Weiterhin können die katalytisch aktiven Metalle beispielsweise nach dem Verfahren von DE-A 2 519 817, EP-A 1 477 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung mit einem Salz oder Komplex der zuvor genannten Metalle erzeugt werden.
Sowohl die Aktivierung der Fällungskatalysatoren als auch der Trägerkatalysatoren kann auch in situ zu Beginn der Reaktion durch den anwesenden Wasserstoff erfolgen, bevorzugt werden diese Katalysatoren jedoch vor ihrer Verwendung separat aktiviert.
Als Trägermaterialien können im allgemeinen die Oxide des Aluminiums und Titans, Zirkoniumdioxid, Siliciumdioxid, Tonerden, z.B. Montmorillonite, Silikate wie Magnesium- oder Aluminiumsilikate, Zeolithe wie ZSM-5 oder ZSM-10-Zeolithe, sowie Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren einsetzbaren Heterogenkatalysatoren dienen. Als für im erfindungsgemäßen Verfahren einsetzbaren Heterogenkatalysatoren seien die folgenden beispielhaft genannt:
Kobalt auf Aktivkohle, Kobalt auf Siliciumdioxid, Kobalt auf Aluminiumoxid, Rhenium auf Aktivkohle, Rhenium auf Siliciumdioxid, Rhenium/Zinn auf Aktivkohle, Rhenium/Platin auf Aktivkohle, Kupfer auf Aktivkohle, Kupfer/Siliciumdioxid, Kupfer, Aluminiumoxid, Kupferchromit, Bariumkupferchromit, Kupfer/Aluminiumoxid/Manganoxid, Kupfer/Aluminiumoxid/Zinkoxid sowie die Katalysatoren gemäß DE-A 3 932 332, US-A 3 449 445, EP-A 44 444, EP-A 147 219, DE-A 3 904 083, DE-A 2 321 101, EP-A 415 202, DE-A 2 366 264, EP 0 552 463 und EP-A 100 406.
Bevorzugte Katalysatoren enthalten mindestens eines der Metalle Kupfer, Mangan, Kobalt, Chrom, Palladium, Platin, Kobalt oder Nickel. Besonders bevorzugt sind Kupfer, Kobalt, Palladium, Platin oder Rhenium. Werden nur Ester hydriert, enthält der Hydrierkatalysator bevorzugt Kupfer.
Für das erfindungsgemäße Verfahren ist es im allgemeinen nicht kritisch, unter welchen Bedingungen bezüglich Temperatur und Druck die Hydrierung durchgeführt wird.

Im allgemeinen liegt die Hydriertemperatur zwischen 100 und 300°C und der Hydrierdruck zwischen 1 und 30 kPa (10 und 300 bar).

Durch die erfindungsgemäße Dosierung der bestimmten basischen Verbindungen, auch unterstöchiometrisch, bezogen auf die eingeschleppten Verunreinigungen, kann einerseits die Acidifizierung, andererseits die strukturelle Umwandlung des Katalysators verhindert oder zumindest stark verzögert werden. Dies äußert sich in der Verbesserung der Selektivität, Erhöhung der Katalysatorstandzeit und, in einer noch nicht eindeutig verstandenen Art und Weise, auch in einer Erhöhung des Umsatzes.
Die bei der Hydrierung gewonnenen Alkohole sind gesuchte Verbindungen, z.B. für Lösungsmittel, Zwischenprodukte oder Vorprodukte für Kunststoffe wie Polyurethane oder Polyester. Das erfindungsgemäße Verfahren wird in den folgenden Beispielen näher erläutert, aber nicht eingeschränkt. Die Analytik der Reaktionsausträge erfolgte gaschromatographisch.

### Beispiel 1

An 25 ml eines CuO (70 %)/Zno (25 %)/Al₂O₃ (5. %)-Katalysators (Fällen einer wäßrigen Lösung von Natriumaluminat und Zink(II)-nitrat-Hexahydrat mit wäßriger Natriumcarbonatlösung, Abfiltrieren des resultierenden Zno- und Al₂O₃-haltigen Niederschlages, Aufschlämmen des Niederschlags mit einer wäßrigen, Kupfer(II)nitrat-Trihydrat und Zink(II)nitrat-Hexahydrat enthaltenden Lösung, Fällen mit wäßriger Natriumcarbonatlösung, Filtrieren, Waschen, Trocknen und Kalzinieren des erhaltenen Niederschlages sowie Verformen des kalzinierten Pulvers zu Tabletten), der zuvor im Wasserstoffstrom bei 180°C aktiviert worden war, wurden ca. 20 g/h einer 50 %igen ethanolischen Lösung von 2-Cyclododecylidenpropionsäureethylester (hergestellt aus 2-Brompropionsäureethylester und Cyclododecan), der noch 7 ppm Halogen (nachgewiesen als Cl) enthielt bei 220°C/220 bar hydriert. Der Gehalt an 2-Cyclododecylpropanol (Moschus-Riechstoff) im Hydrieraustrag lag bei 70 % (ethanolfrei gerechnet). Der Umsatz lag nach ca. 8 h bei ca. 93 % (Selektivität 75 %). Danach wurden in den Zulauf 50 ppm Natriummethanolat eingemischt. Daraufhin stieg der Gehalt an Wertprodukt auf ca. 77 %, der Umsatz auf 96 % (Selektivität 80 %).

### Beispiel 2

An 2,5 1 eines Cu/Al/Mn-Katalysators der Firma Süd-Chemie /T 4489), der zuvor im Wasserstoffstrom bei 180°C aktiviert worden war, wurde ein Gemisch (hergestellt wie in DE-A 19 607 954 im Beispiel, Stufen 1 - 4 beschrieben) aus überwiegend Adipinsäuredimethylester und 6-Hydroxycapronsäuremethylester hydriert (Zulauf 1 kg, Reaktortemperatur 205 bis 220°C, Druck- 25 kPa (250 bar)). Im Hydrierfeed befanden sich ca. 1 ppm Halogenverbindungen nachgewiesen als C1). Zu Beginn der Hydrierung lag das Nebenprodukt Hexandioldiether im Hydrieraustrag bei 0 %. Nach 6 Versuchstagen konnte der Ether nachgewiesen werden. Er stieg bis zum 16. Versuchstag stetig bis auf 0,8 % an (Gehalt an 1,6-Hexandiol im Austrag 27 %, Restgehalte an Adipinsäuredimethylester, 3,6 %, 6-Hydroxycapronsäuremethylester 3,2 %). Danach wurden über eine separaten Zulauf 500 ppm Natriumethanolat, bezogen auf den Hydrierfeed, in Methanol gelöst, in den Reaktor eingebracht. Es erfolgte keine weitere Etherbildung mehr (Gehalt an 1,6-Hexandiol im Austrag 30 %, Restgehalt an Adipinsäuredimethylester 1,1 %, 6-Hydroxycapronsäuremethylester 2,6 %. Während der Fahrweise ohne Na-Zusatz lag der Gehalt an Mn im Hydrieraustrag bei 5 ppm. Während der Na-Dosierung sank der Mn-Austrag unter 3 ppm ab.

## Patentansprüche

1. Verfahren zur katalytischen Hydrierung von Carbonsäuren oder deren Anhydriden oder Estern zu Alkoholen mit Wasserstoff an heterogenen Katalysatoren, die hydrierend wirkende Elemente der Gruppen 6, 7, 8, 9, 10 und 11 sowie gegebenenfalls der Gruppen 2, 14 und 15 des Periodensystems der Elemente enthalten, oder aus diesen bestehen, in flüssiger Phase bei Temperaturen von 100 bis 300°C und Drücken von 1 bis 30 kPa (10 bis 300 bar), **dadurch gekennzeichnet, daß** der Hydrierwasserstoff und/oder der Hydrierfeed Halogenverbindungen enthalten und man in einem geeigneten Lösungsmittel als separaten Strom in die Hydrierung selbst oder in den Hydrierfeed, bezogen auf den flüssigen Hydrierfeed, 1 bis 3000 ppm einer basischen Alkali- oder Erdalkaliverbindung ausgewählt aus der Gruppe bestehend aus den Hydroxiden, Carbonaten, Carboxylaten oder Alkoholaten zusetzt.

2. Verfahren gemäß Anspruch 1, **dadurch gekennzeichnet, daß** man als basische Alkaliverbindungen basische Natrium- oder Kaliumverbindungen verwendet.

3. Verfahren gemäß Anspruch 1, **dadurch gekennzeichnet, daß** man die basische Alkali- oder Erdalkaliverbindungen in Mengen von 5 bis 600 ppm, bezogen auf das flüssige Hydrierfeed, zusetzt.

4. Verfahren gemäß Anspruch 1, **dadurch gekennzeichnet, daß** man Dicarbonsäuren mit 4 bis 6 Kohlenstoffatomen oder deren Anhydride oder Ester zu den entsprechenden Diolen hydriert.

5. Verfahren gemäß Anspruch 1, **dadurch gekennzeichnet, daß** man einen Hydrierkatalysator verwendet, der mindestens eines der Elemente Kupfer, Mangan, Chrom, Palladium, Platin, Kobalt oder Nickel enthält.

6. Verfahren gemäß Anspruch 1, **dadurch gekennzeichnet, daß** der Hydrierkatalysator mindestens eines der Elemente Kupfer, Kobalt, Palladium, Platin oder Rhenium enthält oder daraus besteht.

7. Verfahren gemäß Anspruch 1, **dadurch gekennzeichnet, daß** man Ester in Gegenwart von Katalysatoren hydriert, die Kupfer enthalten oder daraus bestehen.

8. Verfahren gemäß Anspruch 1, **dadurch gekennzeichnet, daß** man Ester von Dicarbonsäuren mit 4 bis 6 C-Atomen an Kupferkatalysatoren unter Zusatz von 5 bis 600 ppm basischer Natrium- oder Kaliumverbindungen zu den entsprechenden Diolen hydriert.

## Claims

1. A process for the catalytic hydrogenation of carboxylic acids or anhydrides or esters thereof to alcohols with hydrogen over heterogeneous catalysts which comprise or consist of hydrogenating elements from groups 6, 7, 8, 9, 10 and 11 and, where appropriate, from groups 2, 14 and 15 of the Periodic Table of the Elements, in the liquid phase at from 100 to 300°C and from 1 to 30 MPa (10 to 300 bar), wherein the hydrogenation hydrogen and/or the hydrogenation feed comprise halogen compounds and which comprises adding, in a suitable solvent as a seperate stream, from 1 to 3000 ppm, based on the liquid hydrogenation feed, of a basic alkali metal compound or alkaline earth metal compound selected from the group consisting of hydroxides, carbonates, carboxylates or alkoxides to the hydrogenation itself or to the hydrogenation feed.

2. A process as claimed in claim 1, wherein the basic alkali metal compounds used are basic sodium or potassium compounds.

3. A process as claimed in claim 1, which comprises adding the basic alkali metal compound or alkaline earth metal compound in amounts of from 5 to 600 ppm, based on the liquid hydrogenation feed.

4. A process as claimed in claim 1, which comprises hydrogenating dicarboxylic acids having from 4 to 6 carbon atoms or anhydrides or esters thereof to the corresponding diols.

5. A process as claimed in claim 1, which comprises using a hydrogenation catalyst which comprises at least one of the elements copper, manganese, chromium, palladium, platinum, cobalt or nickel.

6. A process as claimed in claim 1, wherein the hydrogenation catalyst comprises or consists of at least one of the elements copper, cobalt, palladium, platinum or rhenium.

7. A process as claimed in claim 1, which comprises hydrogenating esters in the presence of catalysts which comprise or consist of copper.

8. A process as claimed in claim 1, which comprises hydrogenating esters of dicarboxylic acids having from 4 to 6 carbon atoms on copper catalysts with addition of from 5 to 600 ppm of basic sodium or potassium compounds to the corresponding diols.

## Revendications

1. Procédé d'hydrogénation par voie catalytique d'acides carboxyliques ou de leurs anhydrides ou esters pour obtenir des alcools avec de l'hydrogène, en présence de catalyseurs hétérogènes contenant ou se composant d'éléments favorisant l'hydrogénation des groupes 6, 7, 8, 9, 10 et 11, et éventuellement des groupes 2, 14 et 15 de ; la Classification Périodique des Eléments, en phase liquide, à des températures allant de 100°C à 300°C et sous des pressions allant de 1 à 30 MPa (10 à 300 bars), **caractérisé en ce que** l'hydrogène d'hydrogénation et/ou la charge d'hydrogénation contiennent des composés halogénés, et **en ce que** l'on ajoute lors de l'hydrogénation elle-même ou dans la charge d'hydrogénation, de 1 à 3000 ppm, par rapport à la charge d'hydrogénation liquide, d'un composé basique de métal alcalin ou alcalino-terreux choisi dans le groupe formé par les hydroxydes, les carbonates, les carboxylates ou les alcoolates, dans un solvant approprié, sous forme de courant séparé.

2. Procédé selon la revendication 1, **caractérisé en ce que** l'on utilise, en tant que composés basiques de métal alcalin, des composés basiques du sodium ou du potassium.

3. Procédé selon la revendication 1, **caractérisé en ce que** l'on utilise les composés basiques de métal alcalin ou alcalino-terreux à raison de 5 à 600 ppm, par rapport à la charge d'hydrogénation liquide.

4. Procédé selon la revendication 1, **caractérisé en ce que** l'on hydrogène des acides dicarboxyliques ayant de 4 à 6 atomes de carbone, ou leurs anhydrides ou esters, pour obtenir les diols correspondants.

5. Procédé selon la revendication 1, **caractérisé en ce que** l'on met en oeuvre un catalyseur d'hydrogénation contenant au moins un des éléments parmi le cuivre, le manganèse, le chrome, le palladium, le platine, le cobalt ou le nickel.

6. Procédé selon la revendication 1, **caractérisé en ce que** le catalyseur d'hydrogénation contient ou se compose d'au moins un des éléments parmi le cuivre, le cobalt, le palladium, le platine ou le rhénium.

7. Procédé selon la revendication 1, **caractérisé en ce que** l'on hydrogène des esters en présence de catalyseurs contenant ou se composant de cuivre.

8. Procédé selon la revendication 1, **caractérisé en ce que** l'on hydrogène des esters d'acides dicarboxyliques ayant de 4 à 6 atomes de carbone, en présence de catalyseurs à base de cuivre, en ajoutant de 5 à 600 ppm de composés basiques du sodium ou du potassium pour obtenir les diols correspondants.
